(19) Europäisches Patentamt
European Patent Office
Office européen des brevets

(11) EP 0 839 135 B1

(12) **EUROPÄISCHE PATENTSCHRIFT**

(45) Veröffentlichungstag und Bekanntmachung des Hinweises auf die Patenterteilung:
**10.10.2001 Patentblatt 2001/41**

(51) Int Cl.⁷: **C07D 217/20**, C07F 17/02, B01J 31/28

(21) Anmeldenummer: **96924872.3**

(86) Internationale Anmeldenummer:
**PCT/EP96/02923**

(22) Anmeldetag: **03.07.1996**

(87) Internationale Veröffentlichungsnummer:
**WO 97/03052 (30.01.1997 Gazette 1997/06)**

(54) **VERFAHREN ZUR HERSTELLUNG VON OPTISCH AKTIVEM 1-(P-METHOXYBENZYL)-1,2,3,4,5,6,7,8-OCTAHYDROISOCHINOLIN**

PROCESS FOR PREPARING OPTICALLY ACTIVE 1-(P-METHOXYBENZYL)-1,2,3,4,5,6,7,8-OCTAHYDROISOQUINOLINE

PROCEDE DE PREPARATION DE 1-(P-METHOXYBENZYL)-1,2,3,4,5,6,7,8-OCTAHYDROISOQUINOLEINE OPTIQUEMENT ACTIVE

(84) Benannte Vertragsstaaten:
**AT BE CH DE DK ES FI FR GB IE IT LI NL PT SE**

(30) Priorität: **11.07.1995 CH 202595**
**05.03.1996 CH 57096**

(43) Veröffentlichungstag der Anmeldung:
**06.05.1998 Patentblatt 1998/19**

(73) Patentinhaber: **F. HOFFMANN-LA ROCHE AG**
**4070 Basel (CH)**

(72) Erfinder: **WERBITZKY, Oleg**
**CH-3930 Visp (CH)**

(56) Entgegenhaltungen:

| | |
|---|---|
| **EP-A- 0 176 856** | **EP-A- 0 307 168** |
| **EP-A- 0 564 406** | **EP-A- 0 612 758** |

- **JOURNAL OF ORGANOMETALLIC CHEMISTRY, Bd. 413, Nr. 1-3, 7.August 1991, LAUSANNE CH, Seiten 295-302, XP000564639 TAMIO HAYASHI ET AL: "Asymmetric synthesis catalysed by chiral ferrocenylphosphine-transition metal complexes"**
- **BULLETIN OF THE CHEMICAL SOCIETY OF JAPAN, Bd. 53, Nr. 4, April 1980, TOKYO JP, Seiten 1138-1151, XP002015945 TAMIO HAYASHI ET AL: "Asymmetric synthesis catalysed by chiral ferrocenylphosphine-transition metal complexes.I.Preparation of chiral ferrocenylphosphines" in der Anmeldung erwähnt**

EP 0 839 135 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft ein Verfahren zur Herstellung von 1-(*p*-Methoxybenzyl)-1,2,3,4,5,6,7,8-octahydroisochinolin der Formel

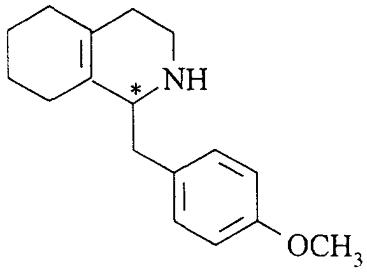

I

in optisch aktiver Form durch asymmetrische Hydrierung.

Sie betrifft weiterhin ein neues Salz des 1-(*p*-Methoxybenzyl)-3,4,5,6,7,8-hexahydroisochinolins und ein Verfahren zu dessen Herstellung.

Sie betrifft ausserdem ein neues chirales Diphosphin mit Ferrocenstruktur und dessen Verwendung zur Herstellung von Katalysatoren für die asymmetrische Hydrierung und die aus dem Diphosphin erhältlichen Iridium-Phosphin-Komplexe.

1-(*p*-Methoxybenzyl)-1,2,3,4,5,6,7,8-octahydroisochinolin (I) ist ein Zwischenprodukt für die Synthese des antitussiven Wirkstoffs Dextromethorphan und des analgetischen Wirkstoffs Levorphanol.
Für eine gezielte Herstellung des wirksamen Enantiomeren von Dextromethorphan wird I in der (*S*)-(-)-Konfiguration benötigt, für die Synthese von Levorphanol in der (*R*)-(+)-Konfiguration. Ein klassisches Verfahren zur Gewinnung dieser Stereoisomeren ist die Racematspaltung, die in diesem Fall auch ohne Einsatz von optisch aktiven Hilfsreagenzien möglich ist (DE-A 34 36 179). Der Hauptnachteil fast aller Racematspaltungen liegt jedoch darin, dass mindestens die Hälfte der eingesetzten Substanz in Form des "unerwünschten" Enantiomeren als Abfall entsorgt werden muss, wenn nicht ausnahmsweise auch hierfür ein mengenmässig vergleichbarer Bedarf existiert.
Im vorliegenden Fall ist auch eine Racemisierung möglich, so dass das unerwünschte Enantiomer als Racemat zurückgeführt werden kann und theoretisch keine Verluste auftreten (O. Schnider et al., *Heiv. Chim. Acta* **1954,** *37,* 710; A. Brossi, O. Schnider, *Heiv. Chim. Acta* **1956,** *39,* 1376; HU 170 924). Diese Methode ist jedoch ziemlich umständlich.

Eine wesentlich bessere Strategie ist die gezielte Synthese durch eine stereoselektive Reaktion, ausgehend von einer prochiralen Vorstufe. Es ist bekannt, dass *N*-Acyl-1-benzyliden-1,2,3,4,5,6,7,8-octahydroisochinoline an der exocyclischen Doppelbindung mit chiralen Ruthenium-Phosphin-Komplexen stereoselektiv ("asymmetrisch") hydriert werden können. (JP-A 05/092 958). Dieses Verfahren hat jedoch den Nachteil, dass ein *N*-acyliertes Produkt erhalten wird, dessen Acylgruppe in einem weiteren Syntheseschritt wieder abgespalten werden muss. Ausserdem entstehen die Benzylidenverbindungen ihrerseits als E/Z-Isomerengemische, wovon jeweils nur das Z-Isomere für die stereoselektive Hydrierung brauchbar ist.

Aufgabe der vorliegenden Erfindung war daher, ein Verfahren bereitzustellen, das direkt die Titelverbindung in guter optischer Reinheit liefert und einfach durchzuführen ist.

Erfindungsgemäss wird die Aufgabe durch das Verfahren gemäss Patentanspruch 1 gelöst.

Es wurde gefunden, dass unter Verwendung von optisch aktiven Iridium-Phosphin-Komplexen als Katalysatoren das 1-(*p*-Methoxybenzyl)-3,4,5,6,7,8-hexahydroisochinolin der Formel

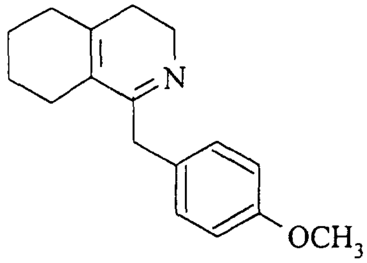

II

oder ein Salz davon direkt asymmetrisch zu den *S*- oder *R*-Enantiomeren der Titelverbindung hydriert werden kann.

Hierbei hängt es von der Art und Konfiguration des Katalysators ab, welches Enantiomer bevorzugt gebildet wird und wie hoch die optische Ausbeute ist. Als optisch aktive Iridium-Phosphin-Komplexe können im Prinzip alle chiralen Komplexe des Iridiums in niedriger Oxidationszahl mit mehrzähnigen chiralen Phosphinen als Liganden eingesetzt werden, die mit Wasserstoff koordinieren können und katalytisch aktiv sind. Für die Hydrierung können sowohl neutrale als auch kationische Iridium-Phosphin-Komplexe als Katalysatoren eingesetzt werden. Diese Katalysatoren können auch *in situ* hergestellt werden. Hierbei wird der aktive Katalysator direkt in der Hydrierungsreaktion durch Ligandenaustausch aus einem Vorläufer-Komplex des Iridiums, dem entsprechenden chiralen Liganden und Wasserstoff gebildet. Als Vorläufer-Komplex kann beispielsweise ein Komplex der allgemeinen Formel $[Ir_2(L_C)_2Cl_2]$, worin $L_C$ ein $C_{4-12}$-Dien bedeutet, eingesetzt werden. In diesem Fall wird ein neutraler Komplex erhalten. Wird dagegen beispielsweise ein Komplex der allgemeinen Formel $[Ir(L_C)_2]^+BF_4^-$, worin $L_C$ die oben genannte Bedeutung hat, eingesetzt, so bildet sich ein kationischer Komplex.

Vorzugsweise werden vorgebildete kationische Komplexe der allgemeinen Formel

$$[IrL_CL_P]^+ A^- \qquad III$$

eingesetzt, die mit Wasserstoff die eigentliche katalytisch aktive Species bilden. $L_C$ steht hierbei für ein $C_{4-12}$-Dien, $L_P$ für ein chirales zweizähniges Phosphin und $A^-$ für ein Anion. Mit zweizähnigen Phosphinen sind hier nicht nur Diphosphine gemeint, sondern auch Phosphine, die ein von Phosphor verschiedenes zweites koordinationsfähiges Atom enthalten, wie beispielsweise Amino-phosphine.

Das Anion $A^-$ ist vorzugsweise ein nicht oder nur schwach koordinierendes Anion wie beispielsweise Tetrafluorborat, Hexafluorphosphat, Hexafluorantimonat, Perchlorat, Phosphat, Acetat, Trifluoracetat, Trifluormethansulfonat oder Toluol-4-sulfonat.

Als Dien $L_C$ kann beispielsweise Norbornadien oder vorzugsweise 1,5-Cyclooctadien enthalten sein.

Beispiele für zweizähnige chirale Phosphinliganden $L_P$ sind:

2,4-Bis(diphenylphosphino)pentan (BDPP),
2-[Phenyl-(3-sulfophenyl)phosphino]-4-(diphenylphosphino)-pentan (BDPP-S),
2,3-Bis(diphenylphosphino)butan (Chiraphos),
4,5-Bis(diphenylphosphino-methyl)-2,2-dimethyl-1,3-dioxolan (DIOP),
2,2'-Bis(diphenylphosphino)-1,1'-binaphthalin (BINAP),
1-*tert*-Butoxycarbonyl-4-diphenylphosphino-2-(diphenylphosphinomethyl)pyrrolidin (BPPM),
2,3-Bis(diphenylphosphino)bicyclo[2.2.1]hept-5-en (Norphos),
1,2-Bis-(2,5-dimethylphospholano)benzol (Me-DUPHOS),
1-Benzyl-3,4-bis(diphenylphosphino)pyrrolidin (Deguphos) oder
Bis(dimethylphosphino)-cyclohexan (BDPPMC).

Je nachdem, welches der beiden Enantiomeren des zweizähnigen chiralen Phosphinliganden eingesetzt wird, kann gezielt die *R*- oder *S*-Form von I hergestellt werden.

Die kationischen Komplexe (III) können beispielsweise dadurch hergestellt werden, dass ein Komplex der allgemeinen Formel $[Ir_2(L_C)_2Cl_2]$, worin $L_C$ ein $C_{4-12}$-Dien bedeutet, mit dem gewünschten zweizähnigen chiralen Phosphinliganden und anschliessend mit einem löslichen Silbersalz, welches das gewünschte Anion $A^-$ enthält, umgesetzt wird. Hierbei wird das in $[Ir_2(L_C)_2Cl_2]$, koordinierte Chlorid als unlösliches Silberchlorid ausgefällt.

Vorzugsweise wird als Phosphinligand $L_P$ ein Ferrocenylphosphin der allgemeinen Formel

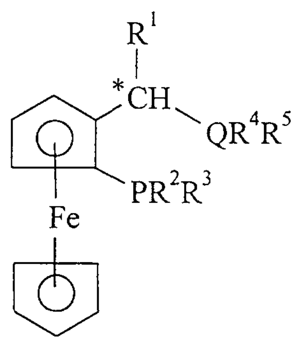

IV

eingesetzt. Hierin steht Q für Stickstoff oder Phosphor und $R^1$ für eine $C_{1-4}$-Alkylgruppe. $R^2$ bis $R^5$ sind jeweils unabhängig voneinander $C_{1-6}$-Alkyl, $C_{3-7}$-Cycloalkyl oder Phenyl, welches gegebenenfalls einen oder mehrere Substituenten wie beispielsweise Methyl, Trifluormethyl oder Methoxy tragen kann.

Besonders bevorzugt sind Phosphinliganden IV, in denen $R^1$ Methyl ist.

Ebenfalls besonders bevorzugt sind Phosphinliganden IV, in denen Q Phosphor ist.

Desgleichen sind besonders bevorzugt Phosphinliganden IV, in welchen $R^2$ und $R^3$ gleich sind und jeweils eine Phenylgruppe oder eine substituierte Phenylgruppe, beispielsweise 4-Methoxy-3,5-dimethylphenyl, bedeuten.

Weiterhin besonders bevorzugt sind Phosphinliganden IV, in denen $R^4$ und $R^5$ gleich sind und $C_{1-4}$-Alkyl, Cyclohexyl oder gegebenenfalls substituiertes Phenyl bedeuten.

Zu den Ferrocenylphosphinen der Formel IV zählen beispielsweise:

(*R*)-1-[(*S*)-2-(Diphenylphosphino)ferrocenyl]ethyl-di-*tert*-butylphosphin (Q = P, $R^1 = CH_3$, $R^2 = R^3 = C_6H_5$, $R^4 = R^5$ = *t*-Bu) [(*R*,*S*)-PPF-P*t*$Bu_2$],

(*R*)-1-[(*S*)-2-(Diphenylphosphino)ferrocenyl]ethyl-dicyclohexyl-phosphin (Q = P, $R^1 = CH_3$, $R^2 = R^3 = C_6H_5$, $R^4 = R^5$ = Cyclohexyl) [(*R*,*S*)-PPF-$PCy_2$],

(*R*)-1-[(*S*)-2-(Diphenylphosphino)ferrocenyl]ethyl-diphenylphosphin (Q = P, $R^1 = CH_3$, $R^2 = R^3 = R^4 = R^5 = C_6H_5$) [(*R*,*S*)-PPF-$PPh_2$],

(*R*)-1-{(*S*)-2-[Bis(4-methoxyphenyl)phosphino]ferrocenyl}ethyl-di-*tert*-butylphosphin (Q = P, $R^1 = CH_3$, $R^2 = R^3$ = *p*-$CH_3OC_6H_4$, $R^4 = R^5$ = *t*-Bu) [(*R*,*S*)-MeOPPF-P*t*$Bu_2$],

(*R*)-1-{(*S*)-2-[Bis(4-trifluormethylphenyl)phosphino]ferrocenyl}ethyl-di-*tert*-butylphosphin (Q = P, $R^1 = CH_3$, $R^2 = R^3$ =*p*-$CF_3C_6H_4$, $R^4 = R^5$ = *t*-Bu) [(*R*,*S*)-$CF_3$PPF-P*t*$Bu_2$],

(*R*)-1-[(*S*)-2-(Di-*p*-tolylphosphino)ferrocenyl]ethyl-di-*tert*-butylphosphin (Q = P, $R^1 = CH_3$, $R^2 = R^3$ =*p*-$CH_3C_6H_4$, $R^4 = R^5$ = *t*-Bu) [(*R*,*S*)-MePPF-P*t*$Bu_2$],

(*R*)-1-[(*S*)-2-(Diphenylphosphino)ferrocenyl]ethyl-bis(4-methoxy-3,5-dimethylphenyl)-phosphin (Q = P, $R^1 = CH_3$, $R^2 = R^3 = C_6H_5$, $R^4 = R^5$ = 4-methoxy-3,5-dimethylphenyl) [(*R*,*S*)-PPF-$PMOD_2$],

*N*,*N*-Dimethyl-{(*R*)-1-[(*S*)-2-(diphenylphosphino)ferrocenyl]ethylamin} (Q = N, $R^1 = R^4 = R^5 = CH_3$, $R^2 = R^3 = C_6H_5$) [(*R*,*S*)-PPFA],

sowie deren Antipoden.

Diese Ferrocenylphosphine sind teils bekannt aus EP-A 0 564 406, EP-A 0 612 758 und T. Hayashi et al., *Bull. Chem. Soc. Jpn.* **1980,** *53,* 1138-1151, teils analog zu den dort beschriebenen Verbindungen erhältlich.

Besonders gute Ergebnisse wurden mit (*R*)-1-{(*S*)-2-[Bis(4-methoxy-3,5-dimethylphenyl)-phosphino]ferrocenyl}ethyl-di-*tert*-butylphosphin (Q = P, $R^1 = CH_3$, $R^2 = R^3$ = 4-methoxy-3,5-dimethylphenyl, $R^4 = R^5$ = *t*-Bu) [(*R*,*S*)-MODPF-P*t*$Bu_2$] als Phosphinligand IV erzielt. Dieses Diphosphin und sein Antipode sind neu und ebenfalls Gegenstand der vorliegenden Erfindung. Sie können durch Umsetzung des entsprechenden Enantiomeren des bekannten *N*,*N*-Dimethyl-1-ferrocenyl-ethylamins mit *n*-Butyllithium und Bis-(4-methoxy-3,5-dimethyl-phenyl)-chlorphosphin zu den entsprechenden *N*,*N*-Dimethyl-1-{2-[bis(4-methoxy-3,5-dimethylphenyl)phosphino]ferrocenyl}ethylaminen und anschliessenden Austausch der Dimethylamino-Gruppe mit Di-*tert*-butylphosphin hergestellt werden.

Von den aus(*R*)-1-{(*S*)-2-[Bis(4-methoxy-3,5-dimethylphenyl)phosphino]ferrocenyl}ethyl-di-*tert*-butylphosphin und seinem Antipoden erhältlichen Iridium-Phosphin-Komplexen sind diejenigen bevorzugt, die durch Umsetzung mit einem Komplex der allgemeinen Formel $[Ir_2(L_C)_2Cl_2]$, worin $L_C$ ein $C_{4-12}$-Dien, vorzugsweise 1,5-Cyclooctadien oder Norbornadien, bedeutet, und anschliessend mit einem Silbersalz aus der Gruppe Silbertetrafluorborat, -hexafluorphosphat, -hexafluorantimonat, -perchlorat, -acetat, -trifluoracetat, -trifluormethansulfonat oder -toluol-4-sulfonat erhältlich sind.

Besonders bevorzugt sind hierbei diejenigen Iridium-Phosphin-Komplexe, bei denen $L_C$ 1,5-Cyclooctadien und das Silbersalz Silbertetrafluorborat ist.

Die asymmetrische Hydrierung wird vorteilhaft bei einer Temperatur von -20 °C bis 100 °C, vorzugsweise von 10 °C bis 40 °C, und einem Druck von 1 bar bis 200 bar, vorzugsweise von 10 bar bis 100 bar, durchgeführt.

Als Lösungsmittel eignen sich beispielsweise aromatische Kohlenwasserstoffe wie Benzol oder Toluol, Ether wie Diethylether, Tetrahydrofuran oder Dioxan, chlorierte Kohlenwasserstoffe wie Dichlormethan oder Dichlorethan, Alkohole wie Methanol, Ethanol oder Isopropylalkohol, Ester wie Ethylacetat oder Butylacetat sowie Gemische dieser Lösungsmittel untereinander oder mit Wasser. Vorzugsweise wird Toluol im Gemisch mit Methanol oder Wasser (2 Phasen) eingesetzt.

Die Stereoselektivität der Hydrierung kann gegebenenfalls durch Zusätze erhöht werden. Hierzu zählen beispielsweise anorganische oder organische Säuren wie Phosphorsäure, Salzsäure, Bromwasserstoffsäure, Iodwasserstoffsäure, Essigsäure oder Trifluoressigsäure, Amine wie Triethylamin, Phosphine wie Triphenylphosphin, oder quartäre Ammonium- oder Phosphoniumverbindungen wie Tetraethyl- oder Tetra-n-butylammoniumfluorid, -chlorid, -bromid, -iodid oder -hydroxid, die entsprechenden quartären Ammoniumsalze des Succinimids, oder die analogen Phosphoniumsalze.

Das Ausgangsmaterial 1-(*p*-Methoxybenzyl)-3,4,5,6,7,8-hexahydroisochinolin ist wenig stabil und disproportioniert leicht. Es wird daher vorzugsweise in Form eines Salzes eingesetzt. Besonders bevorzugt ist das saure Salz der Phosphorsäure, nämlich das 1-(*p*-Methoxybenzyl)-3,4,5,6,7,8-hexahydroisochinolinium-dihydrogenphosphat der Formel

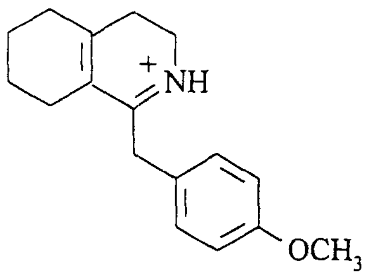

$H_2PO_4^-$ V

Diese neue Verbindung ist ebenfalls Gegenstand der Erfindung. Sie ist in kristalliner Form erhältlich, lagerstabil und einfach herzustellen. Aufgrund des amphoteren Charakters des Dihydrogenphosphations wirkt sie gleichzeitig als Puffer und gewährleistet so die Einhaltung eines optimalen pH-Wertes bei der Hydrierung.

Das Salz wird vorteilhaft hergestellt, indem das aus US-A 4 496 762 bekannte *N*-[2-(Cyclohexen-1-yl)ethyl]-p-methoxyphenylacetamid durch Erhitzen mit Phosphoroxychlorid in einer Bischler-Napieralski-Reaktion cyclisiert und anschliessend in einem organischen Lösungsmittel mit Orthophosphorsäure umgesetzt wird. Als organisches Lösungsmittel eignet sich hier beispielsweise Ethanol, Aceton, Toluol oder Gemische dieser Lösungsmittel, gegebenenfalls mit einem Zusatz von wenig Wasser. Die Kristallisation des Salzes kann durch Animpfen wesentlich verbessert werden. Es ist ebenfalls wichtig, die Salzbildung möglichst rasch nach der Cyclisierung durchzuführen, um eine Zersetzung der freien Base zu vermeiden.

Die nachfolgenden Beispiele verdeutlichen die Ausführung der Erfindung. Beschrieben ist jeweils nur die Reaktion mit einem der beiden Enantiomeren des Katalysators. Selbstverständlich kann bei Verwendung des anderen Enantiomeren unter den gleichen Reaktionsbedingungen das Produkt mit entgegengesetzter Konfiguration erhalten werden.

**Beispiele**

**Beispiel 1**

**1-(*p*-Methoxybenzyl)-3,4,5,6,7,8-hexahydroisochinolin**

In 230 ml Toluol wurden 27,3 g (0,1 mol) *N*-[2-(Cyclohexen-1-yl)ethyl]-*p*-methoxyphenylacetamid und 91,4 g (0,596 mol) Phosphoroxychlorid unter Argon 30 min auf 100 °C erhitzt. Anschliessend wurde das Gemisch am Rotationsverdampfer eingeengt, der Rückstand (46 g) zweimal mit 70 ml Petrolether gewaschen und dann in 270 ml Dichlormethan aufgelöst. Die Lösung wurde bei 0 °C unter kräftigem Rühren zu 270 ml einer 12%igen wässrigen Ammoniaklösung gegeben. Die organische Phase wurde mit 90 ml Wasser gewaschen, über Natriumsulfat getrocknet und eingedampft. Es wurden 27,0 g eines viskosen gelb-braunen Öls erhalten, welches sofort für die Hydrierung eingesetzt wurde.

| | |
|---|---|
| $^1H$ NMR ($CDCl_3$) $\delta$ = | 7,1 (d, 2H, Aryl-H); |
| | 6,8 (d, 2H, Aryl-H); |
| | 3,8 (s, 3H, $OCH_3$); |
| | 3,6 (s, 2H, $CH_2Ar$); |
| | 3,5 (t, 2H, $CH_2N$); |
| | 2,1-2,0 (m, 4H); |
| | 1,9 (m, 2H); |
| | 1,6-1,5 (m, 4H) |

**Beispiel 2**

**1-(*p*-Methoxybenzyl)-3,4,5,6,7,8-hexahydroisochinolinium-dihydrogenphosphat**

In 100 ml Ethanol wurden 27,0 g rohes 1-(*p*-Methoxybenzyl)-3,4,5,6,7,8-hexahydroisochinolin (hergestellt nach Beispiel 1) gelöst. Die Lösung wurde mit Kristallen von 1-(*p*-Methoxybenzyl)-3,4,5,6,7,8-hexahydroisochinolinium-dihydrogenphosphat angeimpft und unter Rühren und Kühlung mit einer Lösung von 12,68 g 85%iger Orthophosphorsäure in 20 ml Ethanol versetzt. Die Lösung wurde auf 0 °C gekühlt und filtriert. Der Niederschlag wurde mit Ethanol gewaschen und getrocknet.
Ausbeute: 23,19 g (65,6%)
Schmp.: 128,9 °C

$^1$H NMR ($CDCl_3$) δ = 7,25 (d, 2H, Aryl-H);
7,02 (d, 2H, Aryl-H);
4,02 (s, 2H, $CH_2$Ar);
3,85 (s, 3H, $OCH_3$)
3,65 (t, 2H, $CH_2$N);
2,55 (t, 2H);
2,4 (m, 2H);
2,3 (m, 2H)
1,7-1,6 (m, 4H).

$^{13}$C NMR ($D_2O$, int. Standard Dioxan) δ = 179,0 (s);
161,1 (s);
159,7 (s);
131,8 (d);
125,7 (s);
124,6 (s);
115,4(d);
56,3 (q);
40,4 (t);
31,9(t);
27,6 (t);
24,2 (t);
22,2 (t);
21,4(t).

**Beispiel 3**

**Herstellung des Katalysators (III, $L_C$ = η$^4$-1,5-Cyclooctadien, $L_P$ = IV, Q = P, $R^1$ = $CH_3$, $R^2$ = $R^3$ = $C_6H_5$, $R^4$ = $R^5$ = *t*-Bu, $A^-$ = $BF_4^-$)**

Unter Argon wurde 1 g (1,49 mmol) Di-μ-chloro-bis(η$^4$-1,5-cyclooctadien)diiridium(1) in 27 ml Dichlormethan gelöst. Zu dieser Lösung wurden 1,65 g (3,13 mmol) (*R*)-1-[(*S*)-2-(Diphenylphosphino)ferrocenyl]ethyl-di-*tert*-butylphosphin gegeben. Die hellrote Lösung wurde mit 0,592 g (2,98 mmol) Silbertetrafluorborat in 9 ml Aceton versetzt. Das Reaktionsgemisch wurde 1 h bei Raumtemperatur gerührt, das ausgefallene Silberchlorid abfiltriert und das Filtrat im Vakuum eingeengt. Der rotbraune Rückstand wurde gewaschen und im Vakuum getrocknet.
Ausbeute: 2,74 g (99%)

$^1$H NMR ($CDCl_3$) δ = 8,25 (dd, 2H);
7,75 (m, 3H);
7,5-7,4 (m, 5H);
5,5 (br. m, 1H);
5,4 (br. m, 1H);
4,7 (m, 1H);
4,5 (m, 1H);
4,3 (m, 1H);
3,9 (br. m, 1H);

3,7 (s, 5H);
3,4 (br. m, 1H);
3,15 (br. m, 1H);
2,2 (dd, 3H);
2,2-2,0 (br. m, 2H);
1,7 (d, 9H); 1,3 (d, 9H);
1,9-1,2 (br. m, 6H).

| | |
|---|---|
| $^{31}$P NMR ($CDCl_3$, 160 MHz) δ = | 61,95 (d, P*t*$Bu_2$); |
| | -9,97 (d, $PPh_2$). |

**Beispiel 4**

**(*S*)-(-)-1-(*p*-Methoxybenzyl)-1,2,3,4,5,6,7,8-octahydroisochinolin**

In einem Autoklaven wurden 35,4 g (0,1 mol) 1-(*p*-Methoxybenzyl)-3,4,5,6,7,8-hexahydroisochinolinium-dihydrogenphosphat (hergestellt nach Beispiel 2) und 62,0 mg (66,6 µmol, entsprechend einem Verhältnis Edukt/Katalysator = 1500) Katalysator (aus Beispiel 3) sowie 38,2 mg (133,4 µmol) Tetrabutylammoniumchlorid vorgelegt. Nachdem die Luft sorgfältig aus dem verschlossenen Autoklaven entfernt und durch Argon ersetzt war, wurden 120 ml sauerstofffreies Toluol und eine ebenfalls sauerstofffreie Lösung von 4,4 g (0,11 mol) Natriumhydroxid in 40 ml Wasser zugegeben. Es wurde Wasserstoff bis zu einem Druck von 70 bar aufgepresst und das Gemisch bei Raumtemperatur 20 h gerührt. Die Wasserphase wurde durch Zugabe von Natriumhydroxid auf pH > 9 eingestellt und nach Trennung der Phasen nochmals mit 100 ml Toluol extrahiert. Die vereinigten organischen Phasen wurden über Natriumsulfat getrocknet und im Vakuum eingeengt. Das Produkt wurde als gelb-braunes viskoses Öl erhalten.

Ausbeute: 23,5 g (91,7%)
$[\alpha]_D^{25}$ =-118 (*c* = 1, MeOH)
Die optische Reinheit wurde durch HPLC an Chiracel® OD (Daicel) bestimmt.
ee = 80%

| | |
|---|---|
| $^1$H NMR ($CDCl_3$) δ = | 7,15 (d, 2H, Aryl-H); |
| | 6,8 (d, 2H, Aryl-H); |
| | 3,8 (s, 3H, $OCH_3$); |
| | 3,25 (br, d, 1H); |
| | 3,1-2,9 (m, 2H); |
| | 2,75 (m, 1H); |
| | 2,45 (dd, 1H); |
| | 2,2-2,1 (m, 1H); |
| | 2,0-1,5 (m, 9H). |

**Beispiel 5**

**(*S*)-(-)-1-(*p*-Methoxybenzyl)-1,2,3,4,5,6,7,8-octahydroisochinolin (Katalysatorherstellung *in situ*)**

Es wurden Lösungen von 1,28 g (5 mmol) 1-(*p*-Methoxybenzyl)-3,4,5,6,7,8-hexahydroisochinolin in 10 ml sauerstofffreiem Toluol und von 8,4 mg (12,5 µmol) Di-µ-chloro-bis-($\eta^4$-1,5-cyclooctadien)diiridium(I) und 14,9 mg (27,5 µmol) (*R*)-1-[(*S*)-2-(Diphenylphosphino)-ferrocenyl]ethyl-di-*tert*-butylphosphin in 9 ml sauerstofffreiem Toluol und 1 ml sauerstofffreiem Methanol hergestellt. Beide Lösungen wurden über Kanülen in einen sorgfältig von Luft befreiten 50 ml-Autoklaven gegeben. Anschliessend wurde Wasserstoff bis zu einem Druck von 70 bar aufgepresst. Das Reaktionsgemisch wurde 21 h bei Raumtemperatur gerührt und anschliessend im Vakuum eingeengt.
Ausbeute: 1,21 g
$[\alpha]_D^{25}$ =-102(*c*= 1, MeOH)
ee = 82,5% (HPLC)

**Beispiel 6**

***N,N*-Dimethyl-(*R*)-1-{(*S*)-2-[bis(4-methoxy-3,5-dimethylphenyl)phosphino]ferrocenyl}-ethylamin**

Durch Umsetzung von Bis(3,5-dimethyl-4-methoxyphenyl)phosphinigsäure (T. Morimoto et al., *Tetrahedron*

*Lett.* **1989,** *30*, 735) mit Phosphortrichlorid und Destillation des Reaktionsprodukts bei 185 °C/0,2 mbar wurde das Bis-(4-methoxy-3,5-dimethylphenyl)-chlorphosphin hergestellt. In 11 ml *tert*-Butylmethylether wurden unter Argon 1,71 g (5,82 mmol) (*R*)-*N*,*N*-Dimethyl-1-ferrocenylethylamin gelöst. Es wurden 3,5 ml (8,75 mmol) *n*-Butyllithium (2,5 M Lösung in Hexan) zugetropft, dann wurde eine Lösung von 2,94 g (8,73 mmol) Bis-(4-methoxy-3,5-dimethylphenyl)-chlorphosphin in 5 ml *tert*-Butylmethylether zugegeben. Das braune Reaktionsgemisch wurde auf 50 °C erwärmt und weitere 4 h gerührt. Nach dem Abkühlen auf 0 °C wurden 0,35 g $NaHCO_3$ in 7 ml Wasser langsam zugesetzt. Nach Zugabe von 10 ml Dichlormethan wurde das Reaktionsgemisch filtriert und der unlösliche Rückstand nochmals mit 10 ml Dichlormethan nachgewaschen. Die wässrige Phase wurde dreimal mit 5 ml Dichlormethan extrahiert, die vereinigten organischen Phasen mit 5 ml Wasser gewaschen, über Natriumsulfat getrocknet und eingeengt. Das braune ölige Rohprodukt (4,3 g) wurde aus 14 ml Ethanol umkristallisiert.
Ausbeute: 0,98 g (30,2%) brauner Feststoff
$[\alpha]^{25}_{D}$ = -272,4 (*c* = 0,4; $CHCl_3$)

$^1$H NMR ($CDCl_3$, 400 MHz) δ =
1,28 (d, 3H, $CH(NMe_2)CH_3$);
1,8 (s, 6H, $NMe_2$);
2,18 (s, 6H, $PhCH_3$);
2,32 (s, 6H, $PhCH_3$);
3,62 (s, 3H, $OCH_3$);
3,72 (s, 3H, $OCH_3$);
3,85 (s, 1H, $C_5H_3$);
3,92 (s, 5H, $C_5H_5$);
4,06 (q, 1H, $CHNMe_2$);
4,22 (s, 1H, $C_5H_3$);
4,35 (s, 1H, $C_5H_3$);
6,82 (d, 2H, $C_6H_2$);
7,25 (d, 2H, $C_6H_2$).

$^{31}$P NMR ($CDCl_3$, 160 MHz) δ = -23,7

**Beispiel 7**

**(*R*)-1-{(*S*)-2-[Bis(4-methoxy-3,5-dimethylphenyl)phosphino]ferrocenyl}ethyl-di-*tert*-butylphosphin**

In 18 ml Essigsäure wurden unter Argon 0,979 g (1,76 mmol) *N*,*N*-Dimethyl-(*R*)-1-{(*S*)-2-[bis(4-methoxy-3,5-dimethylphenyl)phosphino]ferrocenyl}ethylamin (hergestellt nach Beispiel 6) suspendiert. Dann wurden 0,345 g (2,36 mmol) Di-*tert*-butylphosphin zugegeben und das Reaktionsgemisch 2,5 h bei 80°C gerührt. Die Reaktionslösung wurde im Vakuum eingeengt, und man erhielt 1,3 g eines rot-braunen Öles, welches mit Ethylacetat/Hexan/Ethanol (47,5 : 47,5 : 5) über eine Kieselgel-Säule chromatographiert wurde. Ausbeute: 0,9 g (77%) orangeroter Feststoff
$[\alpha]^{25}_{D}$ =-324,6 (*c* = 0,4; $CHCl_3$)

$^1$H NMR ($CDCl_3$, 400 MHz) δ =
0,97 (d, 9H; *t*Bu);
1,15 (d, 9H; *t*Bu);
1,82 (dd, 3H, $CHP\mathit{t}Bu_2CH_3$);
2,18 (s, 6H, $PhCH_3$);
2,25 (s, 6H, $PhCH_3$);
3,35 (q, 1H, $CHP\mathit{t}Bu_2$);
3,62 (s, 3H, $OCH_3$);
3,72 (s, 3H, $OCH_3$);
3,88 (s, 5H, $C_5H_5$);
3,95 (s, 1H, $C_5H_3$);
4,2 (s, 1H, $C_5H_3$);
4,35 (s, 1H, $C_5H_3$);
6,82 (d, 2H, $C_6H_2$);
7,3 (d, 2H, $C_6H_2$).

$^{31}$P NMR ($CDCl_3$. 160 MHz) δ =
49,3 (d, $^4J_{PP}$ = 44,5 Hz, $P\mathit{t}Bu_2$);
-26,7 (d, $^4J_{PP}$ = 44,5 Hz, $PPh_2$).

**Beispiel 8**

**Herstellung des Katalysators:**

Unter Argon wurden 115 mg (170,8 μmol) Di-μ-chloro-bis($\eta^4$-1,5-cyclooctadien)diiridium(I) in 3,4 ml Dichlormethan gelöst. Zu dieser Lösung wurden bei 0 °C 250 mg (379,6 μmol) (*R*)-1-{(*S*)-2-[Bis(3,5-dimethyl-4-methoxyphenyl)phosphino]ferrocenyl}ethyl-di-*tert*-butyl-phosphin (hergestellt nach Beispiel 7) gegeben. Die hellrote Lösung wurde mit 67,9 mg (341,6 μmol) Silbertetrafluorborat in 1,1 ml Aceton versetzt. Das Reaktionsgemisch wurde 1 h bei 0 °C gerührt, das ausgefallene Silberchlorid abfiltriert und das Filtrat im Vakuum eingeengt. Der rotbraune Rückstand wurde mit Diethylether gewaschen und im Vakuum getrocknet.
Ausbeute: 350 mg (98%)

| | |
|---|---|
| $^1$H NMR ($CDCl_3$, 400 MHz) δ = | 7,95 (d, 2H); |
| | 7,05 (d, 2H); |
| | 5,6 (br. s, 1H); |
| | 5,25 (br. s, 1H); |
| | 4,7 (s, 1H); |
| | 4,5 (s, 1H); |
| | 4,3 (s, 1H); |
| | 3,84 (s, 3H); |
| | 3,82 (s, 3H); |
| | 3,78 (s, 5H); |
| | 3,7(br. s, 1H); |
| | 3,3 (br. s, 2H); |
| | 2,45 (s, 6H); |
| | 2,3 (s, 6H); |
| | 2,2 (dd, 3H); |
| | 2,3-2,1 (br. m, 2H); |
| | 1,65 (d, 9H); |
| | 1,25 (d, 9H); |
| | 2,0-1,5 (br. m, 6H). |

| | |
|---|---|
| $^{31}$P NMR ($CDCl_3$, 160 MHz) δ = | 61,5 (d, P*t*Bu); |
| | -7,45 (d, $PPh_2$). |

**Beispiel 9**

**(*S*)-1-(*p*-Methoxybenzyl)-1,2,3,4,5,6,7,8-octahydroisochinolin**

In einem Autoklaven wurden 3,54 g (10 mmol) 1-(*p*-Methoxybenzyl)-3,4,5,6,7,8-hexahydroisochinoliniumdihydrogenphosphat (hergestellt nach Beispiel 2) und 6,97 mg (6,66 μmol, entsprechend einem Verhältnis Edukt/Katalysator = 1500) Katalysator aus Beispiel 8 sowie 8,68 mg Tetrabutylammoniumbromid vorgelegt. Nachdem die Luft sorgfältig aus dem verschlossenen Autoklaven entfernt und durch Argon ersetzt war, wurden 14 ml sauerstofffreies Toluol und eine ebenfalls sauerstofffreie Lösung von 440 mg Natriumhydroxid in 40 ml Wasser zugegeben. Es wurde Wasserstoff bis zu einem Druck von 70 bar aufgepresst und das Gemisch bei Raumtemperatur 22 h gerührt. Die Wasserphase wurde durch Zugabe von Natriumhydroxid auf pH > 9 eingestellt und nach der Trennung der Phasen nochmals mit 10 ml Toluol extrahiert. Die vereinigten organischen Phasen wurden über Natriumsulfat getrocknet und im Vakuum eingeengt. Das Produkt wurde als gelb-braunes Öl erhalten. Ausbeute: 2,32 g (90,2%)
$[\alpha]_D^{25}$ = -131,7 (*c* = 1, MeOH)
Die optische Reinheit wurde durch HPLC an Chiracel® OD (Daicel) bestimmt.
ee = 89,2%.

**Beispiele 10-24**

Analog zu den Beispielen 4 und 5 wurden die in der folgenden Tabelle 1 zusammengefassten Umsetzungen durchgeführt.
In der Spalte "Edukt" sind die Formelnummern der eingesetzten Edukte angegeben. Die Spalte "P" gibt den verwendeten Vorläufer-Komplex an. (Hierbei steht "A" für Ir(cod)$L_P$ (hergestellt analog zu Beispiel 3) (cod = 1,5-Cyclooctadien)

und "B" für $[Ir_2(cod)_2Cl_2]$.
Die in der Spalte "Ligand" eingetragenen Kurzbezeichnungen entsprechen den in der vorstehenden Aufzählung der Liganden in Klammern angegebenen Abkürzungen.
In der Spalte "E/K" ist das molare Verhältnis Edukt : Katalysator angegeben.
"RT" bedeutet Raumtemperatur.

Die in der Spalte "Zusätze" verwendeten Abkürzungen bedeuten im einzelnen:

| | |
|---|---|
| TBACl = | Tetra-*n*-butylammoniumchlorid |
| TBAF = | Tetra-*n*-butylammoniumfluorid |
| TBAI = | Tetra-*n*-butylammoniumiodid |
| TBASI = | Tetra-*n*-butylammonium, Salz mit Succinimid |
| TBPBr = | Tetra-*n*-butylphosphoniumbromid |
| TEAH = | Tetraethylammoniumhydroxid |

**Tabelle 1**

| Edukt | P | Ligand | E/K | Lösungsmittel | Temp. | *p* [bar] | Zusätze | ee [%] |
|---|---|---|---|---|---|---|---|---|
| 30 mmol V | A | (*R*,*S*)-PPF-P*t*$Bu_2$ | 600 | 80 ml Dioxan/6,3 ml $H_2O$ | RT | 70 | 2,0 mmol TBACl, 24 mmol NaOH, 24 mmol TEAH | 85,4 (*S*) |
| 30 mmol V | A | (*R*,*S*)-PPF-P*t*$Bu_2$ | 1000 | 38 ml Toluol/10 ml $H_2O$ | RT | 10 | 0,12 mmol TBACl, 33 mmol NaOH | 79,1 (*S*) |
| 20 mmol II | A | (*R*,*S*)-PPF-P*t*$Bu_2$ | 800 | 19 ml Toluol/1 ml MeOH | RT | 70 | 6,66 mmol $H_3PO_4$ | 71,1 (*S*) |
| 5 mmol II | B | (*R*,*S*)-PPF-P*t*$Bu_2$ | 200 | 19 ml Toluol/1 ml MeOH | RT | 70 | | 85,2 (*S*) |
| 10 mmol V | A | (*R*,*S*)-PPF-P*t*$Bu_2$ | 3000 | 14 ml Toluol/3,3 ml $H_2O$ | RT | 70 | 11 mmol NaOH, 13,3 mmol TBAI | 35,8 (*S*) |
| 10 mmol V | A | (*R*,*S*)-MODPF-P*t*$Bu_2$ | 1500 | 7 ml Toluol/7 ml Dioxan/3,3 ml $H_2O$ | RT | 70 | 11 mmol NaOH, 13,3 mmol TBAI | 75,8 (*S*) |
| 5 mmol II | B | (+)-Norphos | 100 | 10 ml Toluol/10 ml MeOH | RT | 70 | | 30,7 (*S*) |
| 5 mmol II | B | (*R*,*R*)-Chiraphos | 100 | 2 ml Toluol/18 ml MeOH | RT | 70 | | 38,4 (*R*) |
| 5 mmol II | B | (*R*,*S*)-PPF-$Pcy_2$ | 100 | 20 ml MeOH | RT | 70 | | 53,9 (*R*) |
| 5 mmol II | B | (*R*,*R*)-BDPP-S | 100 | 20 ml MeOH | 0 °C | 70 | | 16,7 (*S*) |
| 5 mmol II | B | (+)-(*S*,*S*)BDPPMC | 100 | 20 ml MeOH | RT | 70 | | 18,9 (*S*) |
| 5 mmol II | B | (+)-(*S*,*S*)BDPPMC | 100 | 20 ml Toluol | RT | 70 | | 16,7 (*R*) |
| 10 mmol V | A | (*R*,*S*)-PPF-P*t*$Bu_2$ | 1000 | 14 ml Toluol, 3,3 ml $H_2O$ | RT | 70 | 11 mmol NaOH, 40 µmol TBPBr | 79,7 (*S*) |
| 10 mmol V | A | (*R*,*S*)-PPF-P*t*$Bu_2$ | 1000 | 14 ml Toluol, 3,3 ml $H_2O$ | RT | 70 | 11 mmol NaOH, 40 µmol TBAF | 82,5 (*S*) |
| 10 mmol V | A | (*R*,*S*)-PPF-P*t*$Bu_2$ | 1000 | 14 ml Toluol, 3,3 ml $H_2O$ | RT | 70 | 11 mmol NaOH, 40 µmol TBASI | 79,6 (*S*) |

## Patentansprüche

1. Verfahren zur Herstellung von optisch aktivem 1-(*p*-Methoxybenzyl)-1,2,3,4,5,6,7,8-octahydroisochinolin der Formel

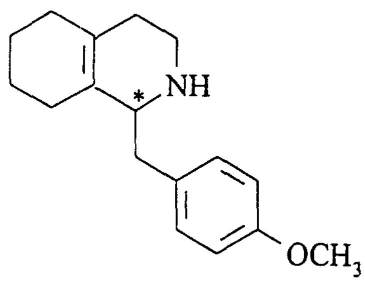

I

**dadurch gekennzeichnet, dass** 1-(*p*-Methoxybenzyl)-3,4,5,6,7,8-hexahydroisochinolin der Formel

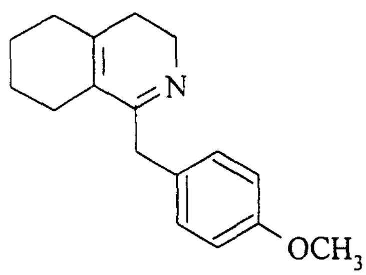

II

oder ein Salz davon in Gegenwart eines katalytisch wirksamen optisch aktiven Iridium-Phosphin-Komplexes asymmetrisch hydriert wird.

2. Verfahren nach Anspruch 1, **dadurch gekennzeichnet, dass** der katalytisch wirksame optisch aktive Iridium-Phosphin-Komplex aus einem Komplex der allgemeinen Formel

$$[IrL_CL_P]^+ A^- \qquad III$$

oder aus dem Reaktionsprodukt von $[Ir(L_C)_2Cl_2]$ oder $[Ir(L_C)_2]^+ BF_4^-$ und $L_P$, worin jeweils $L_C$ ein $C_{4-12}$-Dien, $L_P$ ein chirales zweizähniges Phosphin und $A^-$ ein Anion bedeutet, gebildet wird.

3. Verfahren nach Anspruch 2, **dadurch gekennzeichnet, dass** $L_C$ 1,5-Cyclooctadien bedeutet.

4. Verfahren nach Anspruch 2, **dadurch gekennzeichnet, dass** $L_P$ ein Ferrocenylphosphin der allgemeinen Formel

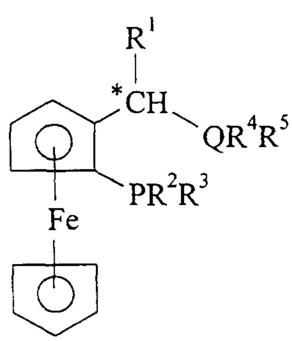

IV

ist, worin Q Stickstoff oder Phosphor, $R^1$ eine $C_{1-4}$-Alkylgruppe und $R^2$ bis $R^5$ unabhängig voneinander $C_{1-6}$-Alkyl, $C_{3-7}$-Cycloalkyl oder gegebenenfalls substituiertes Phenyl bedeuten.

5. Verfahren nach Anspruch 4, **dadurch gekennzeichnet, dass** $R^1$ Methyl ist.

6. Verfahren nach Anspruch 4 oder 5, **dadurch gekennzeichnet, dass** Q Phosphor ist.

7. Verfahren nach einem der Ansprüche 4 bis 6, **dadurch gekennzeichnet, dass** $R^2$ und $R^3$ gleich sind und Phenyl oder substituiertes Phenyl bedeuten.

8. Verfahren nach Anspruch 7, **dadurch gekennzeichnet, dass** $R^2$ und $R^3$ 4-Methoxy-3,5-dimethylphenyl bedeuten.

9. Verfahren nach einem der Ansprüche 4 bis 8, **dadurch gekennzeichnet, dass** $R^4$ und $R^5$ gleich sind und $C_{1-4}$-Alkyl, Cyclohexyl oder gegebenenfalls substituiertes Phenyl bedeuten.

10. Verfahren nach den Ansprüchen 4 bis 9, **dadurch gekennzeichnet, dass** als Ligand $L_P$ (*R*)-1-{(*S*)-2-[Bis(4-methoxy-3,5-dimethylphenyl)phosphino]ferrocenyl}ethyl-di-*tert*-butylphosphin oder dessen Antipode eingesetzt wird.

11. Verfahren nach einem oder mehreren der Ansprüche 1 bis 10, **dadurch gekennzeichnet, dass** das 1-(*p*-Methoxybenzyl)-3,4,5,6,7,8-hexahydroisochinolin in Form des Dihydrogenphosphats der Formel

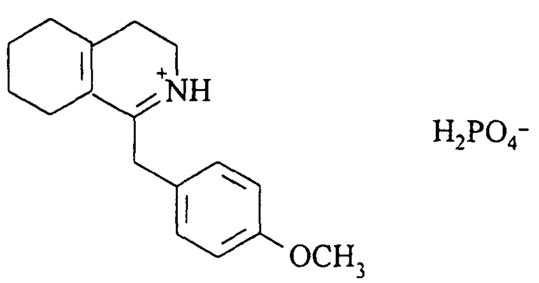

V

eingesetzt wird.

12. 1-(*p*-Methoxybenzyl)-3,4,5,6,7,8-hexahydroisochinolinium-dihydrogenphosphat (V).

13. Verfahren zur Herstellung von 1-(*p*-Methoxybenzyl)-3,4,5,6,7,8-hexahydroisochinolinium-dihydrogenphosphat, **dadurch gekennzeichnet, dass** *N*-[2-(Cyclohexen-1-yl)-ethyl]-*p*-methoxyphenylacetamid durch Erhitzen mit Phosphoroxychlorid cyclisiert und anschliessend mit Orthophosphorsäure in einem organischen Lösungsmittel umgesetzt wird.

14. (*R*)-1-{(*S*)-2-[Bis(4-methoxy-3,5-dimethylphenyl)phosphino]ferrocenyl}ethyl-di-*tert*-butylphosphin.

15. (*S*)-1-{(*R*)-2-[Bis(4-methoxy-3,5-dimethylphenyl)phosphino]ferrocenyl}ethyl-di-*tert*-butylphosphin.

16. Verwendung von 1-{2-[Bis(4-methoxy-3,5-dimethylphenyl)phosphino]ferrocenyl}ethyl-di-*tert*-butylphosphin gemäss Ansprüchen 14 und 15 zur Herstellung optisch aktiver Katalysatoren für die asymmetrische Hydrierung.

17. Iridium-Phosphin-Komplex, erhältlich durch Umsetzung eines Komplexes der allgemeinen Formel $[Ir_2(L_C)_2Cl_2]$, worin $L_C$ ein $C_{4-12}$-Dien, vorzugsweise 1,5-Cyclooctadien oder Norbornadien, bedeutet, mit einem 1-{2-[Bis(4-methoxy-3,5-dimethylphenyl)phosphino]ferrocenyl}ethyl-di-*tert*-butylphosphin gemäss Ansprüchen 14 und 15 und einem Silbersalz aus der Gruppe Silbertetrafluorborat, -hexafluorphosphat, -hexafluorantimonat, -perchlorat, -acetat, -trifluoracetat, -trifluormethansulfonat oder -toluol-4-sulfonat.

18. Iridium-Phosphin-Komplex nach Anspruch 17, **dadurch gekennzeichnet, dass** $L_C$ 1,5-Cyclooctadien und das Silbersalz Silbertetrafluorborat ist.

## Claims

1. Process for the preparation of optically active 1-(ρ-methoxybenzyl)-1,2,3,4,5,6,7,8-octahydroisoquinoline of the formula

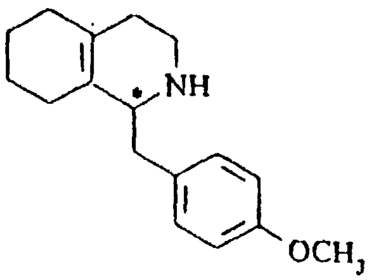

I

**characterised in that** 1-(ρ-methoxybenzyl)-3,4,5,6,7,8-hexahydroisoquinoline of the formula

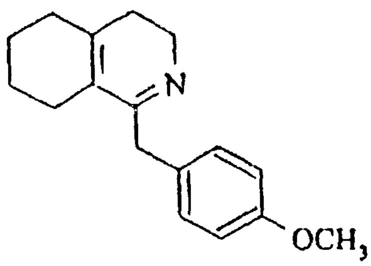

II

or a salt thereof is asymmetrically hydrogenated in the presence of a catalytically acting and optically active iridium-phosphine complex.

2. Process according to claim 1, **characterised in that** the catalytically acting, optically active iridium-phosphine complex is formed from a complex of the general formula

$$[IrL_C L_P]^+ A^- \qquad III$$

or from the reaction product of $[Ir(\mathbf{L_C})_2Cl_2]$ or $[Ir_2(\mathbf{L_C})_2]^+BF^-_4$ and $\mathbf{L_P}$, wherein in each case $\mathbf{L_C}$ denotes a $C_{4}$-$_{12}$ diene, $\mathbf{L_P}$ denotes a chiral bidentate phosphine, and $A^-$ denotes an anion.

3. Process according to claim 1, **characterised in that** $\mathbf{L_C}$ denotes 1,5-cyclooctadiene.

4. Process according to claim 2, **characterised in that** $\mathbf{L_P}$ is a ferrocenyl phosphine of the general formula

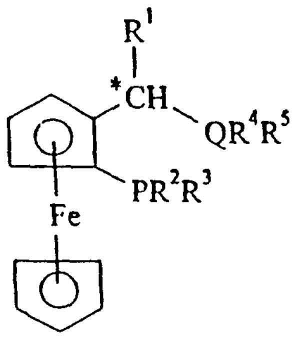

IV

wherein Q denotes nitrogen or phosphorus, $R^1$ denotes a $C_{1-4}$ alkyl group, and $R^2$ to $R^5$ denote independently of one another $C_{1-6}$ alkyl, $C_{3-7}$ cycloalkyl or optionally substituted phenyl.

**5.** Process according to claim 4, **characterised in that** $R^1$ is methyl.

**6.** Process according to claim 4 or 5, **characterised in that** Q is phosphorus.

**7.** Process according to any one of claims 4 to 6, **characterised in that** $R^2$ and $R^3$ are identical and denote phenyl or substituted phenyl.

**8.** Process according to claim 7, **characterised in that** $R^2$ and $R^3$ denote 4-methoxy-3,5-dimethylphenyl.

**9.** Process according to any one of claims 4 to 8, **characterised in that** $R^4$ and $R^5$ are identical and denote $C_{1-4}$-alkyl, cyclohexyl or optionally substituted phenyl.

**10.** Process according to claims 4 to 9, **characterised in that** (*R*)-1-{(*S*)-2-[bis(4-methoxy-3,5-dimethylphenyl)phosphino]ferrocenyl}ethyl-di-*tert*-butylphosphine or its antipode is used as ligand $\mathbf{L_P}$.

**11.** Process according to one or more of claims 1 to 10, **characterised in that** 1-(ρ-methoxybenzyl)-3,4,5,6,7,8-hexahydroisoquinoline is used in the form of the dihydrogen phosphate of the formula

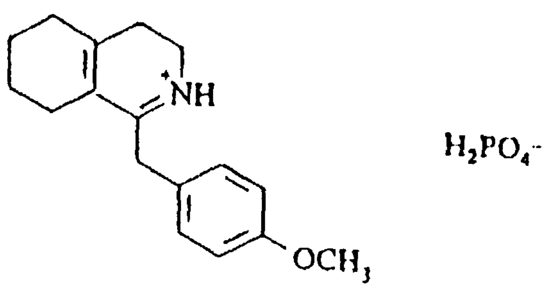

V

**12.** 1-(ρ-methoxybenzyl)-3,4,5,6,7,8-hexahydroisoquinolinium dihydrogen phosphate (V) .

**13.** Process for the preparation of 1-(ρ-methoxybenzyl)-3,4,5,6,7,8-hexahydroiso-quinolinium dihydrogen phosphate, **characterised in that** *N*-[2-(cyclohexen-1-yl)ethyl]-*p*-methoxyphenyl acetamide is cyclised by heating with phosphorus oxychloride and is then reacted with orthophosphoric acid in an organic solvent.

**14.** (*R*)-1-{(*S*)-2-[bis(4-methoxy-3,5-dimethylphenyl)phosphino]ferrocenyl}-ethyl-di- *tert*-butylphosphine.

**15.** (*S*)-1- {(*R*)-2-[bis(4-methoxy-3,5-dimethylphenyl)phosphino]ferrocenyl}-ethyl-di-*tert*-butylphosphine.

**16.** Use of 1-(2-[bis(4-methoxy-3,5-dimethylphenyl)phosphino]ferrocenyl}-ethyl-di-*tert*-butylphosphine according to

claims 14 and 15 for the preparation of optically active catalysts for asymmetrical hydrogenation.

**17.** Iridium-phosphine complex obtainable by reacting a complex of the general formula $[Ir_2(\mathbf{L_C})_2Cl_2]$, wherein $\mathbf{L_C}$ denotes a $C_{4}$-$_{12}$ diene, preferably 1,5-cyclooctadiene or norbornadiene, with a 1-{2-[bis(4-methoxy-3,5-dimethylphenyl)phosphino]ferrocenyl}ethyl-di-*tert*-butylphosphine according to claims 14 and 15, and a silver salt from the group comprising silver tetrafluoroborate, hexafluorophosphate, hexafluoroantimonate, perchlorate, acetate, trifluoroacetate, trifluoromethanesulfonate or toluene-4-sulfonate.

**18.** Iridium-phosphine complex according to claim 17, **characterised in that $\mathbf{L_C}$** is 1,5-cyclooctadiene and the silver salt is silver tetrafluoroborate.

**Revendications**

**1.** Procédé de préparation de 1-(*p*-méthoxybenzyl)-1,2,3,4,5,6,7,8-octahydroisoquinoléine de formule :

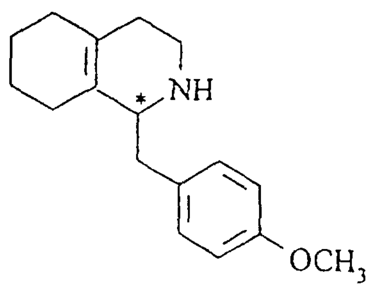

(I)

**caractérisé en ce qu'**on hydrogène de façon asymétrique la 1-(*p*-méthoxybenzyl)-1,2,3,4,5,6,7,8-octahydroisoquinoléine de formule :

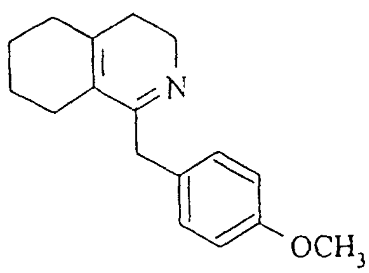

(II)

ou un de ses sels en présence d'un complexe d'iridium-phosphine optiquement actif, catalytiquement efficace.

**2.** Procédé selon la revendication 1, **caractérisé en ce que** le complexe d'iridium-phosphine optiquement actif catalytiquement efficace est formé d'un complexe de formule générale :

$$[IrL_cL_p]^+ A^- \qquad (III)$$

ou du produit de réaction de $[Ir(L_c)_2Cl_2]$ ou $[Ir(Lc)_2]^+BF^-_4$ et de $L_p$, où à chaque fois $L_c$ représente un diène en $C_4$ à $C_{12}$, $L_p$ une phosphine bidentée chirale et $A^-$ un anion.

**3.** Procédé selon la revendication 2, **caractérisé en ce que** Lc représente le 1,5-cyclooctadiène.

**4.** Procédé selon la revendication 2, **caractérisé en ce que** $L_p$ représente une ferrocénylphosphine de formule générale :

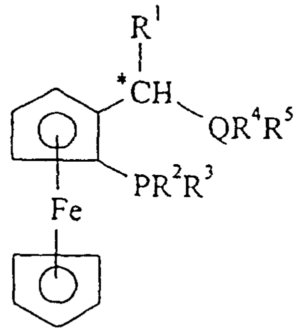

(IV)

dans laquelle Q représente l'azote ou le phosphore, $R^1$ représente un groupe alkyle en $C_1$ à $C_4$ et $R^2$ à $R^5$ représentent indépendamment l'un de l'autre un alkyle en $C_1$ à $C_6$, un cycloalkyle en $C_3$ à $C_7$ ou un phényle éventuellement substitué.

5. Procédé selon la revendication 4, **caractérisé en ce que** $R^1$ est un méthyle.

6. Procédé selon la revendication 4 ou 5, **caractérisé en ce que** Q est le phosphore.

7. Procédé selon l'une des revendications 4 à 6, **caractérisé en ce que** $R^2$ et $R^3$ sont identiques et représentent un phényle ou un phényle substitué.

8. Procédé selon la revendication 7, **caractérisé en ce que** $R^2$ et $R^3$ représentent un 4-méthoxy-3,5-diméthylphényle.

9. Procédé selon l'une des revendications 4 à 8, **caractérisé en ce que** $R^4$ et $R^5$ sont identiques et représentent un alkyle en $C_1$ à $C_4$, un cyclohexyle ou un phényle éventuellement substitué.

10. Procédé selon les revendications 4 à 9, **caractérisé en ce qu'**on utilise comme ligand $L_p$ la *(R)*-1-{*(S)*-2-[Bis-(4-méthoxy-3,5-diméthylphényl)-phosphino]-ferrocényl}-éthyl-di-*tert*-butylphosphine ou son antipode.

11. Procédé selon une ou plusieurs des revendications 1 à 10, **caractérisé en ce qu'**on utilise la 1-(*p*-méthoxybenzyl)-3,4,5,6,7,8-hexahydroisoquinoléine sous la forme du phosphate diacide de formule :

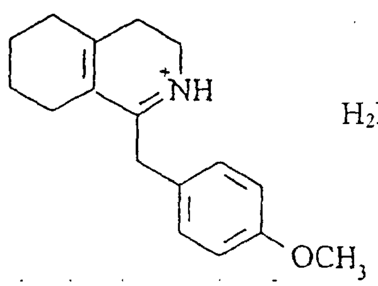

$H_2PO_4^-$

(V)

12. Phosphate diacide de 1-(*p*-méthoxybenzyl)-3,4,5,6,7,8-hexahydroisoquinolinium (V).

13. Procédé de préparation de phosphate diacide de 1-(*p*-méthoxybenzyl)-3,4,5,6,7,8-hexahydroisoquinolinium, **caractérisé en ce qu'**on cyclise le *N*-[2-(cyclohexène-1-yl)-éthyl]-*p*-méthoxyphénylacétamide par chauffage avec de l'oxychlorure de phosphore, puis **en ce qu'**on le fait réagir avec de l'acide orthophosphorique dans un solvant organique.

14. *(R)*-1-{*(S)*-2-[Bis(4-méthoxy-3,5-diméthylphényl)-phosphino]-ferrocényl}-éthyl-di-*tert*-butylphosphine.

15. *(S)*-1-{*(R)*-2-[Bis-(4-méthoxy-3,5-diméthylphényl)-phosphino]-ferrocényl}-éthyl-di-*tert*-butylphosphine.

16. Utilisation de 1-{2-[Bis-(4-méthoxy-3,5-diméthylphényl)-phosphino]-ferrocényl}-éthyl-di-*tert*-butylphosphine selon

les revendications 14 et 15 pour la préparation de catalyseurs optiquement actifs pour l'hydrogénation asymétrique.

**17.** Complexe d'iridium-phosphine que l'on peut obtenir par réaction d'un complexe de formule générale $[I_r(L_c)_2Cl_2]$, où $L_c$ représente un diène en $C_4$ à $C_{12}$, de préférence un 1,5-cyclooctadiène ou un norbornadiène, avec une 1-{2-[Bis-(4-méthoxy-3,5-diméthylphényl)-phosphino]-ferrocényl}-éthyl-di-*tert*-butylphosphine selon les revendications 14 et 15 et un sel d'argent du groupe constitué par le tétrafluoroborate d'argent, l'hexafluorophosphate d'argent, l'hexafluoroantimonate d'argent, le perchlorate d'argent, l'acétate d'argent, le trifluoroacétate d'argent, le trifluorométhanesulfonate d'argent ou le toluène-4-sulfonate d'argent.

**18.** Complexe d'iridium-phosphine selon la revendication 17, **caractérisé en ce que** $L_C$ représente le 1,5-cyclooctadiène et le sel d'argent est le tétrafluoroborate d'argent.